(19) Europäisches Patentamt
European Patent Office
Office européen des brevets

(11) **EP 4 285 835 A1**

(12) **EUROPEAN PATENT APPLICATION**

(43) Date of publication:
**06.12.2023 Bulletin 2023/49**

(21) Application number: **22177153.8**

(22) Date of filing: **03.06.2022**

(51) International Patent Classification (IPC):
***A61B 8/08*** *(2006.01)*     ***A61B 5/00*** *(2006.01)*

(52) Cooperative Patent Classification (CPC):
**A61B 8/5207; A61B 5/0095; A61B 5/7267; A61B 8/5261; G06N 3/08;** A61B 5/7264; G06N 3/045; G06N 3/048

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Helmholtz Zentrum München - Deutsches Forschungszentrum für Gesundheit und Umwelt (GmbH)**
**85764 Neuherberg (DE)**
• **iThera Medical GmbH**
**81379 München (DE)**

(72) Inventors:
• **JÜSTEL, Dominik**
**80796 München (DE)**
• **DEHNER, Christoph**
**80339 München (DE)**
• **MORSCHER, Stefan**
**83530 Schnaitsee (DE)**
• **ZAHND, Guillaume**
**80689 München (DE)**
• **LONGO, Antonia**
**80538 München (DE)**

(74) Representative: **Linsmeier, Josef**
**Wallinger Ricker Schlotter Tostmann**
**Patent- und Rechtsanwälte Partnerschaft mbB**
**Zweibrückenstrasse 5-7**
**80331 München (DE)**

(54) **METHODS AND SYSTEM FOR OPTOACOUSTIC AND/OR ULTRASONIC IMAGING, RECONSTRUCTING OPTOACOUSTIC AND/OR ULTRASONIC IMAGES AND TRAINING AN ARTIFICIAL NEURAL NETWORK PROVIDED THEREFOR**

(57) The invention relates to a method and corresponding system for optoacoustic and/or ultrasonic imaging, a method for reconstructing optoacoustic and/or ultrasonic images and a method for training an artificial neural network provided therefor, the training method comprising: a) providing a model of the imaging apparatus, the model characterizing a relation between i) a spatial distribution of acoustic sources emitting and/or reflecting acoustic waves and ii) signals generated by detection elements of the imaging apparatus upon detecting the acoustic waves, b) providing several training signal sets, each training signal set comprising a plurality of training signals which were i) generated by the imaging apparatus upon imaging objects and/or ii) obtained by simulating an imaging of objects by the imaging apparatus based on the model of the imaging apparatus, c) reconstructing, based on the model of the imaging apparatus, several training image data sets from the training signal sets, each training image data set comprising image data relating to an optoacoustic and/or ultrasonic image of an object, and d) training the artificial neural network, which comprises an input layer and an output layer, the training comprising i) inputting the training signal sets at the input layer, ii) obtaining, for each inputted training signal set, an output image data set which is outputted at the output layer, and iii) comparing each output image data set with the training image data set which was reconstructed from the respectively inputted training signal set.

Fig. 1

**Description**

The present invention relates to a method and corresponding system for optoacoustic and/or ultrasonic imaging, a method for reconstructing optoacoustic and/or ultrasonic images and a method for training an artificial neural network provided therefor.

Optoacoustic imaging, also referred to as "photoacoustic" imaging, requires reconstruction of an initial pressure distribution ($p_0$) that is induced by laser illumination of biological tissue by suitable image reconstruction algorithms. Clinical in-vivo applications of optoacoustic imaging require high-quality images that resolve details in the tissue contrast, a fast reconstruction of the initial pressure distribution and image display to enable live feedback to the user for dynamic imaging (that is, operations where the user needs real-time image display to position the probe), live tuning of the speed of sound parameter to enable dynamic focusing of the image for different imaged tissue types, and compatibility of the live image reconstruction with high data rates and high image resolution to enable optimal data usage without loss of image quality.

Hybrid optoacoustic and ultrasound (OPUS) systems are configured to acquire both optoacoustic (OA) and ultrasound (US) signals. The associated image reconstruction requires a reconstruction of both the initial optoacoustic pressure distribution ($p_0$) in optoacoustic imaging and the reflection coefficient distribution ($\Gamma$) in ultrasound imaging. The relation between both these quantities and the recorded OA and US data depends on acoustic properties of the tissue, particularly on the speed of sound distribution (SoS). Optimal usage of OPUS data requires a reconstruction of the acoustic properties (SoS, mechanical density) simultaneously with the two unknowns ($p_0$, $\Gamma$). Commercial usage of the imaging data requires realization of the mapping (US data, OA data) $\mapsto$ (SoS, $\Gamma$, $p_0$) in real time (preferably at least 25 frames per second) in the system hardware to enable live feedback to the system user on the system monitor.

It is an object of present disclosure to provide a method and corresponding system for optoacoustic and/or ultrasonic imaging, a method for reconstructing optoacoustic and/or ultrasonic images and a method for training an artificial neural network provided therefor which are improved in view of at least a part of the above-mentioned needs.

This object is achieved by a method for training an artificial neural network for reconstructing optoacoustic and/or ultrasonic images according to claim 1, a method for reconstructing optoacoustic and/or ultrasonic images according to claim 11, a method for optoacoustic and/or ultrasonic imaging according to claim 12 and a system for optoacoustic and/or ultrasonic imaging according to claim 13.

A first aspect of present disclosure relates to a method for training an artificial neural network for reconstructing optoacoustic and/or ultrasonic images from signals generated by an imaging apparatus for optoacoustic and/or ultrasonic imaging, wherein the method comprises: a) providing a model, which is also referred to as "forward model", of the imaging apparatus, the model characterizing a relation, which is also referred to as "mapping", between i) a spatial distribution, also referred to as "image", of acoustic sources emitting and/or reflecting acoustic waves and ii) signals, also referred to as "sinogram","sinograms" or "data", generated by detection elements of the imaging apparatus upon detecting the acoustic waves, b) providing several training signal sets, also referred to as "training sinograms", each training signal set comprising a plurality of training signals which were i) generated by the imaging apparatus upon imaging objects and/or ii) obtained by simulating an imaging of objects by the imaging apparatus based on the model of the imaging apparatus, c) reconstructing, based on the model of the imaging apparatus, several training image data sets from the training signal sets, each training image data set comprising image data relating to an optoacoustic and/or ultrasonic image of an object, and d) training the artificial neural network, which comprises an input layer and an output layer, the training comprising i) inputting the training signal sets at the input layer, ii) obtaining, for each inputted training signal set, an output image data set which is outputted at the output layer, and iii) comparing each output image data set with the training image data set which was reconstructed from the respectively inputted training signal set.

A second aspect of present disclosure relates to a method for reconstructing an optoacoustic and/or ultrasonic image from a set of signals, which is also referred to as "sinogram" or may be or comprise a "set of sinograms", generated by an imaging apparatus for optoacoustic and/or ultrasonic imaging, the method comprising: inputting the set of signals at an input layer of the artificial neural network which has been trained by the method according to the first aspect, and obtaining at least one optoacoustic and/or ultrasonic image which is outputted at an output layer of the trained artificial neural network.

A third aspect of present disclosure relates to a method for optoacoustic and/or ultrasonic imaging comprising: irradiating an object with electromagnetic and/or acoustic waves and generating a set of signals, which is also referred to as "sinogram" or may be or comprise a "set of sinograms", by detecting acoustic waves emitted and/or reflected by the object in response thereto by means of an imaging apparatus for optoacoustic and/or ultrasonic imaging, and reconstructing an optoacoustic and/or ultrasonic image of the object from the set of signals by the method according to the second aspect.

A fourth aspect of present disclosure relates to a system for optoacoustic and/or ultrasonic imaging comprising an imaging apparatus for optoacoustic and/or ultrasonic imaging, the imaging apparatus comprising an irradiation device configured to irradiate an object with electromagnetic and/or acoustic waves and a detection device configured to generate a set of signals, which is also referred to as "sinogram" or may be or comprise a "set of sinograms", by detecting acoustic waves emitted and/or reflected by the object in response to irradiating the object with the electromagnetic and/or acoustic waves, and a processor configured to reconstruct an optoacoustic and/or ultrasonic image of the object from the set of signals by inputting the set of signals at an input layer of an artificial neural network which has been trained by the method according to the first aspect, and obtaining at least one optoacoustic and/or ultrasonic image which is outputted at an output layer of the trained artificial neural network.

Preferably, in particular in relation to the second to fourth aspect, in the case that the set of signals comprises a single sinogram, a single-wavelength optoacoustic image can be reconstructed therefrom. It is preferred that the set of signals comprises a set of sinograms (i.e. the set of sinograms comprises several sinograms) from which, e.g., multi-wavelength optoacoustic image(s) and/or ultrasonic image(s), including superimposed and/or co-registered and/or "hybrid" optoacoustic image(s) and/or ultrasonic image(s), can be reconstructed or obtained, respectively.

A fifth aspect of present disclosure relates to a computer program product causing a computer, computer system and/or distributed computing environment to execute the method according to i) the first aspect of present disclosure and/or ii) the second aspect of present disclosure and/or iii) the third aspect of present disclosure.

A sixth aspect of present disclosure relates to a computer, computer system and/or distributed computing environment (e.g. a client-server system or storage and processing resources of a computer cloud) comprising means for carrying out the method according to i) the first aspect of present disclosure and/or ii) the second aspect of present disclosure and/or iii) the third aspect of present disclosure.

A seventh aspect of present disclosure relates to a computer-readable storage medium having stored thereon instructions which, when executed by a computer, computer system or distributed computing system, cause same to carry out the method according to i) the first aspect of present disclosure and/or ii) the second aspect of present disclosure and/or iii) the third aspect of present disclosure.

Preferably, within the meaning of present disclosure, the term "acoustic source" relates to any entity contained in an imaged object which emits ultrasound (in response to stimulating the entity with pulsed electromagnetic radiation) and/or which reflects ultrasound (in response to applying ultrasound to the entity).

Preferred aspects of present disclosure are based on the approach of providing a dedicated method for training an artificial neural network, also referred to as "deep learning" and "deep neural network", respectively, and using the trained artificial neural network for reconstructing optoacoustic and/or ultrasonic images from signals generated by an imaging apparatus for optoacoustic and/or ultrasonic imaging. A preferred first implementation, herein also referred to as "DeepMB", relates to a deep learning solution for optoacoustic (OA) image reconstruction, preferably with tunable speed of sound (SoS). A preferred second implementation, herein also referred to as "DeepOPUS", relates to a deep learning solution for simultaneous (synergistic) reconstruction of co-registered optoacoustic (OA) and ultrasonic (US) images, preferably including speed of sound distribution (SoS) retrieval.

Preferably, in the DeepMB implementation a comprehensive dataset of input-output pairs (OA data, SoS) $\mapsto$ (OA image) is provided and/or generated based on i) a precise modelling and simulation of the OA response (also referred to as OA data) of the imaging system (forward model), and ii) an implementation of an iterative model-based image reconstruction methodology. Then, a deep neural network is trained with the afore-mentioned data set. The obtained trained neural network can then be implemented in a system hardware, e.g. via dedicated graphical processing units, and/or used for optoacoustic image reconstruction, wherein a set of optoacoustic signals (sinogram) acquired by an optoacoustic imaging apparatus is inputted at an input layer of the trained neural network, and an optoacoustic image is obtained at an output layer of the trained neural network. In this way, it is possible to reconstruct high-quality OA images with arbitrary content at a high framerate of at least 24 fps and to dynamically ("on-the-fly") change the SoS during imaging.

Similarly, the DeepOPUS implementation preferably includes i) a precise modelling and simulation of the OA and US responses (also referred to as OA and US data, respectively) of the imaging system (forward model), and ii) an implementation of a model-based solution (based on the afore-mentioned forward model) to the inverse problem, i.e., realization of the mapping (US data, OA data) $\mapsto$ (SoS, $\Gamma$, $p_0$), wherein the reflection coefficient distribution $\Gamma$ corresponds to an ultrasonic (US) image, and the initial pressure distribution $p_0$ corresponds to an optoacoustic (OA) image. A comprehensive data set of input-output pairs ((US data, OA data), (SoS, $\Gamma$, $p_0$)) is provided and/or generated, preferably via simulation of US data and OA data, e.g. from a public general feature image database, and reconstruction of (SoS, $\Gamma$, $p_0$) with the method (model-based solution) set forth in the afore-mentioned item ii). A deep neural network is trained with the afore-mentioned data set. The obtained trained neural network can then be implemented in a system hardware, e.g. via dedicated graphical processing units, and/or used for optoacoustic and ultrasonic image reconstruction, wherein a set of optoacoustic and ultrasonic signals (sinograms) acquired by an optoacoustic and ultrasonic imaging apparatus is inputted at an input layer of the trained neural network, and an optoacoustic and ultrasonic image is obtained at an output layer of the trained neural network. In this way, it is possible to properly utilize the combination of OA and US data simultaneously (as opposed to "one after the other") to i) quantify the pixel-wise SoS distribution in the imaged region, ii) correct for reflection artifacts in both OA and US images, obtain a high framerate of at least 24 fps, and iii) improve the image quality via the synergistic effects of OA and US data integration.

In summary, by means of present disclosure the quality of reconstructed OA and/or US images is improved, and high frame rates are achieved. In particular, present disclosure allows for correcting image artifacts in both optoacoustic and ultrasonic images and quantifying and/or dynamically changing the speed of sound distribution in the imaged region.

Preferably, the model characterizes at least one of the following: i) a propagation of the acoustic waves from the acoustic sources towards the detection elements, ii) a response of the detection elements upon detecting the acoustic waves, and/or iii) a noise of the imaging apparatus.

Preferably, characterizing the propagation of the acoustic waves includes at least one of the following: i) an acoustic wave propagation model, which is the same for the propagation of both emitted optoacoustic waves and reflected ultrasound waves, ii) a propagation of the acoustic waves through a medium with an inhomogeneous speed of sound distribution, and/or iii) a reflection of the acoustic waves at one or more reflective interfaces in the medium investigated.

Preferably, at least some of the training signal sets comprise training signals which were obtained (synthesized) by simulating an imaging of objects by the imaging apparatus based on i) the model of the imaging apparatus and ii) initial images of objects which were obtained by any imaging apparatus. For example, the initial images can be photographs, e.g. "real-world images", from arbitrary objects and/or scenes taken by a conventional camera. Alternatively or additionally, the initial images can be medical and/or in-vivo images obtained by a medical and/or tomographic imaging modality, for example a CT, MRT, ultrasound and/or optoacoustic imaging modality.

Preferably, in particular in the DeepMB implementation, reconstructing a training image data set $x^*$ from a training signal set $s$ comprises: i) calculating, based on the model $M$ of the imaging apparatus, several prediction signal sets $M(x)$ from several varying image data sets $x$, ii) calculating, for each of the varying image data sets $x$, a first distance metric $d(M(x), s)$ between the respective prediction signal set $M(x)$ and the training signal set $s$, and iii) determining the image data set $x^*$ for which the first distance metric $d(M(x), s)$ between the respective prediction signal set $M(x)$ and the training signal set $s$ exhibits a minimum, wherein the reconstructed training image data set is the determined image data set $x^*$.

Preferably, in particular in the DeepOPUS implementation, reconstructing at least one training image data set $x^*_{OA}$, $x^*_{US}$, $c^*$ from at least one training signal set $SOA$, $s_{US}$) comprises: i) calculating, based on the model $M_c$ of the imaging apparatus taking into account a propagation of the acoustic waves through a medium with a, in particular predefined or reconstructed, speed of sound distribution $c$, several prediction signal sets $M_c(x_{OA}, x_{US})$ from several varying image data sets $x_{OA}$, $x_{US}$, ii) calculating, for each of the varying image data sets $x_{OA}$, $x_{US}$, a second distance metric $d(M_c(x_{OA}, x_{US}), (s_{OA}, s_{US}))$ between the respective prediction signal set $M_c(x_{OA}, x_{US})$ and the training signal set $s_{OA}$, $s_{US}$, and iii) determining at least one image data set $x^*_{OA}$, $x^*_{US}$, $c^*$ for which the second distance metric $d(M_c(x_{OA}, xus), (s_{OA}, sus))$ between the respective prediction signal set $d(M_c(x_{OA}, xus)$ and the at least one training signal set $s_{OA}$, $s_{US}$ exhibits a minimum, wherein the at least one training image data set is the at least one determined image data set $x^*_{OA}$, $x^*_{US}$, $c^*$. Preferably, the speed of sound distribution $c$ can be an inhomogeneous or homogeneous speed of sound distribution.

Preferably, comparing the output image data set with the respective training image data set $x^*_{OA}$, $x^*_{US}$, $c^*$ comprises determining a loss function which is given by: a third distance metric, in particular a means squared error, between the output image data set, on the one hand, and the respective training image data set $x^*_{OA}$, $x^*_{US}$ and speed of sound distribution $c^*$ reconstructed from the respective training signal set, on the other hand, and/or the first and/or second distance metric which is applied to the output image data set.

Preferably, the at least one artificial neural network is given by i) a single deep neural network or ii) a cascade of multiple (N) deep neural networks.

Preferably, in a so-called one-step process, the training comprises i) inputting the training signal sets $s_{OA}$, $s_{US}$ at the input layer, ii) obtaining, for each inputted training signal set $s_{OA}$, $s_{US}$, both the output image data set $x_{OA}$, $x_{US}$ and an output speed of sound distribution $c$ which are outputted at the output layer, and iii) comparing each output image data set $x_{OA}$, $x_{US}$ and output speed of sound distribution $c$ with the training image data set $x^*_{OA}$, $x^*_{US}$ and, respectively, a training speed of sound distribution $c^*$ which were reconstructed from the respectively inputted training signal set $SOA$, $s_{US}$.

Preferably, in a so-called two-step process, the training comprises i) inputting the training signal sets $s_{OA}$, $s_{US}$ at the input layer, ii) obtaining, for each inputted training signal set $s_{OA}$, $s_{US}$, an output speed of sound distribution $c$ which is outputted at the output layer, and iii) comparing each output speed of sound distribution $c$ with a training speed of sound distribution $c^*$ which was reconstructed from the respectively inputted training signal set $s_{OA}$, $s_{US}$, and subsequently i) inputting the training signal sets $s_{OA}$, $s_{US}$ and the output speed of sound distribution $c$ at the input layer, ii) obtaining, for each inputted training signal set $s_{OA}$, $s_{US}$ and output speed of sound distribution $c$, the output image data set $x_{OA}$, $x_{US}$ which is outputted at the output layer, and iii) comparing each output image data set $x_{OA}$, $x_{US}$ with the training image data set $x^*_{OA}$, $x^*_{US}$ which was reconstructed from the respectively inputted training signal set $SOA$, $s_{US}$.

Preferably, each training signal set may comprise several training sinograms and/or a set of training sinograms (i.e. the set of training sinograms comprises several training sinograms) so as to particularly train the artificial neural network for reconstructing and/or obtaining, e.g., multi-wavelength optoacoustic image(s) and/or ultrasonic image(s), including superimposed and/or co-registered and/or "hybrid" optoacoustic image(s) and/or ultrasonic image(s).

Other preferred and/or alternative aspects and/or embodiments of present disclosure are discussed in the following, in particular with reference to the DeepOPUS and DeepMB implementation.

DeepOPUS

Preferably, the DeepOPUS implementation includes correctly modeling, learning and inferring a mapping between ultrasound and optoacoustic signals (sinogram(s)), on the one hand, and an ultrasound and optoacoustic image and a speed of sound (SoS) distribution, on the other hand: {ultrasound signals, optoacoustic signals} $\rightarrow$ {ultrasound image, optoacoustic image, speed of sound distribution}.

Preferably, the DeepOPUS implementation includes at least one of the following aspects or a combination thereof: 1) providing a forward model that simulates the physics, in particular the acoustic propagation path, and data acquisition of the optoacoustic and ultrasound imaging process, 2) providing an inverse problem solver that reconstructs optoacoustic and ultrasound images as well as the speed of sound distribution from the optoacoustic and ultrasound signal data using the forward model, and 3) providing a deep learning solution that implements the inverse problem solver in real time on the system, where the forward model can be used to generate, preferably synthetic, training data obtained from a, for example real-world, image dataset.

In the following, preferred embodiments of the above-mentioned aspects 1) to 3) are described.

1) The forward model

Preferably, the forward model

a. simulates acoustic wave propagation for both optoacoustic and ultrasound waves with the same acoustic physics model (i.e. the optoacoustic and ultrasound model are compatible, and/or

b. simulates acoustic wave propagation through media with inhomogeneous speed of sound distributions, and/or

c. simulates (at least one) reflection event(s) at reflective interfaces in the medium.

Feature a. is preferred to couple the information contained in the two different OA and US imaging modalities. Features b. and c. are preferred to allow speed of sound inference.

Preferably, the forward model of the acoustic components of the system takes into account different aspects: (i) the physics of acoustic wave propagation, (ii) the conversion of acoustic pressure to electrical signals by the detectors, (iii) the system noise. In summary, it provides a function that maps images (or volumes) of initial pressure/reflectivity data to simulated optoacoustic/ultrasound sinograms.

Regarding (i), the model needs to approximate solutions to the acoustic wave equation

$$\Delta p - \frac{1}{c^2}\frac{\partial^2}{\partial t^2} p = 0,$$

with p the pressure, t the time, and c the speed of sound distribution.

For the optoacoustic part, an initial value problem needs to be solved with $p(x,0) = p_0$ the initial pressure distribution. The second initial condition $d/dt\, p(x,0) = 0$ is a result of the fast optical absorption process in optoacoustics.

For the ultrasound part, the inhomogeneous wave equation needs to be solved with a source term that describes the acoustic excitation of tissue. There are several algorithmic options for solving these problems in a common framework: finite element solvers, pseudo-spectral methods (e.g., k-wave), geometric acoustics solvers (e.g., raytracing).

Regarding (ii), the pressure recorded at the detectors is preferably described by the total impulse response of the system, i.e., the signal generated by an impulse (infinitesimally small acoustic source) located in the field of view of the system. If the system is linear, the impulse response fully characterizes the output of the system. The experimental and computational characterization of the impulse response and its integration into a forward model is described in detail in the following publications, which are incorporated by reference herewith: Chowdhury, K.B., Prakash, J., Karlas, A., Jüstel, D. & Ntziachristos, V. A Synthetic Total Impulse Response Characterization Method for Correction of Hand-Held Optoacoustic Images. IEEE Trans Med Imaging 39, 3218-3230 (2020), and Chowdhury, K.B., Bader, M., Dehner, C., Jüstel, D. & Ntziachristos, V. Individual transducer impulse response characterization method to improve image quality of array-based handheld optoacoustic tomography. Opt Lett 46, 1-4 (2021).

Regarding (iii), noise can preferably be incorporated as random distortions of the data, if a probabilistic model of the noise is available. It can also be integrated from measured pure system noise, for example, when the noise is signal independent and additive. Often noise is not explicitly included into the model and dealt with during the image reconstructions (e.g., with suitable regularization schemes for variational formulations of the inverse problem; see below). Details on electrical noise in optoacoustic imaging can be found in the following publication, which is incorporated by reference herewith: Dehner, C., Olefir, I., Basak Chowdhury, K., Jüstel, D. and Ntziachristos, V. Deep learning based electrical noise removal enables high spectral optoacoustic contrast in deep tissue. arXiv:2102.12960 (2021).

2) The inverse problem solver

Preferably, the inverse problem solver

    a. approaches the inverse problem via a variational formulation of the problem (also called model-based approach), i.e., a distance metric between the data and the prediction of the forward model is minimized to recover the unknowns (namely, ultrasound image, optoacoustic image, speed of sound distribution), and/or

    b. addresses the nonlinearity and potential ill-posedness of the problem, preferably with integration of prior information and other regularization techniques, and with suitable strategies for initial guesses.

Feature a. represents the preferred use of the most powerful framework for inverse problems. Feature b. provides optimal performance of the solver.

Preferably, the inverse problem solver is an implementation of an algorithm that approximates the image/volume data, given the sinogram data acquired in optoacoustic or ultrasound imaging.

Preferably, a very general and powerful approach to inverse problems is the variational formulation of the inverse problem. The idea is to minimize a distance metric between the acquired signals and the prediction of the forward model (see above) over a set of possible solutions. The inverse problem can then be tackled with solvers for optimization problems (e.g., gradient descent methods). If M is the model that maps an image x to a sinogram s, and d is a distance metric for sinograms, then an image reconstruction $x^*$ is, for example, given by

$$x^* = \arg\min_x \quad d(M(x), s).$$

In the case of DeepOPUS, the unknowns - speed of sound distribution c, optoacoustic image $x_{OA}$, and ultrasound image xus - are reconstructed in the same way. The dependence of the model on the speed of sound is indicated by writing $M_c$. Note that the model $M_c$ has two outputs for DeepOPUS: an optoacoustic sinogram $s_{OA}$ and ultrasound sinogram $s_{US}$. The metric d, thus, needs to quantify the distance between the collections of sinograms:

$$(x_{OA}^*,\ x_{US}^*,\ c^*) = \arg\min_{(x_{OA}, x_{US}, c)} \quad d\big(M_c(x_{OA},\ x_{US}), (s_{OA},\ s_{US})\big).$$

Such non-linear minimization problems can be solved with specific solvers for certain choices of d (e.g., non-linear least squares) or more generally with gradient descent methods or Newton's method. If the objective function is convex in the unknowns, then every local minimum is global, such that the latter methods can be used to find a solution. In non-convex situations, a suitable initial guess is necessary to converge to a global minimum.

An additional complication may be ill-posedness, which in present case means the non-uniqueness of the solution. This issue can be addressed by using appropriate assumptions, and incorporate an additional regularization term that enforces well-posedness and therefore unicity of the solution.

3) The deep learning solution

Preferably, for the deep learning solution at least one of the following applies:

    a. During training, the artificial neural network learns the mapping and has as inputs the optoacoustic and ultrasound signal data, and as output the optoacoustic and ultrasound images, and the speed of sound distribution.

    b. The input of the artificial neural network for the training is a synthetic dataset of simulated optoacoustic and ultrasound data, obtained by using the forward model onto a dataset of real-world images. The target reference for the training are the corresponding ground-truth reconstructions of optoacoustic and ultrasound images, together with the speed of sound distribution, obtained by applying the inverse problem solver onto the input training data.

c. When deployed to acquired optoacoustic and ultrasound signal data, the trained artificial neural network applies the learned mapping that has as inputs the acquired optoacoustic and ultrasound signal data, and as output the optoacoustic and ultrasound images, and the speed of sound distribution in real time.

Features a. and b. ensure that a well-defined operator is learned, such that the model generalizes properly to unseen data with minimal dependence on the specifics of the training data set. Feature c. provides the performance which is preferred for real time feedback to the system operator.

The deep learning solution speeds up the solution from the inverse problem solver for on-device application during imaging. It infers the reconstructed OA and US images and the SoS distribution from the recorded OA and US sinograms of a scan.

Preferably, the training data are OA and US sinograms that were synthesized with the forward model (see 1) based on suitable real-world images, and corresponding ground truth reference data (OA and US images, SoS distribution) obtained from the inverse problem solver (see 2).

Preferably, the deep learning solution employs one of the following options as loss function during training:

I. A suitable distance metric (e.g., mean squared error) between the output of the employed deep neural network and the ground truth images and SoS distribution obtained from the inverse problem solver (see 2).

II. All or parts of the distance metrics that are used in the variational formulation of the inverse problem solver in 2, applied to the output of the employed deep neural network.

III. A combination of I and II.

Deep neural network architecture

Preferably, the deep learning solution infers the OA and US images and the SoS distribution either in a one-step or in a two-step process using a single or a cascade of N deep neural networks, respectively. Further details can be found, for example, in the following publication which is incorporated by reference herewith: Hauptmann, A., Lucka, F., Betcke, M., Huynh, N., Adler, J., Cox, B., Beard, P., Ourselin, S. and Arridge, S. Model-Based Learning for Accelerated, Limited-View 3-D Photoacoustic Tomography. IEEE Trans Med Imaging 37, 1382-1393 (2018),

One-step process with single deep neural network:

```
(OA_sinogram, US_sinogram) →DNN1 (OA_image, US_image, SoS_distribution)
One-step process with cascade of N deep neural networks:
(OA_sinogram, US_sinogram, OA_image(init), US_image(init), SoS_distribution(init))
→DNN1(1) (OA_image(1), US_image(1), SoS_distribution(1))
(OA_sinogram, US_sinogram, OA_image(1), US_image(1), SoS_distribution(1))
→DNN1(2) (OA_image(2), US_image(2), SoS_distribution(2))
(OA_sinogram, US_sinogram, OA_image(N-1), US_image(N-1), SoS_distribution(N-
1)) →DNN1(N) (OA_image(N), US_image(N), SoS_distribution(N))
Two-step process with single deep neural network:
(OA_sinogram, US_sinogram) →DNN1 (SoS_distribution)
followed by:
(OA_sinogram, US_sinogram, SoS_distribution) →DNN2 (OA_image, US_image)
Two-step process with cascade of N deep neural networks:
(OA_sinogram, US_sinogram, SoS_distribution(init)) →DNN1(1) (SoS_distribution(1))
(OA_sinogram, US_sinogram, SoS_distribution(1)) →DNN1(2) (SoS_distribution(2))
(OA_sinogram, US_sinogram, SoS_distribution(N-1) →DNN1(N) (SoS_distribu-
tion(N))
followed by
(OA_sinogram, US_sinogram, SoS_distribution, OA_image(init), US_image(init))
→DNN2(1) (OA_image(1), US_image(1))
(OA_sinogram, US_sinogram, SoS_distribution, OA_mage(1), US_image(1))
→DNN2(2) (OA_image(2), US_image(2))
(OA_sinogram, US_sinogram, SoS_distribution, OA_image(N-1), US_image(N-1))
→DNN2(N) (OA_image(N), US_image(N))
```

OA_image$^{(init)}$, US_image$^{(init)}$, SoS_distribution$^{(init)}$ are initial guesses that are preferably derived at random, from the OA and US sinograms, or from further prior information.

If not specified otherwise, "OA_image", "US_image", and "SoS_distribution" correspond to the final version, namely "OA_image$^{(N)}$", "US_image$^{(N)}$", and "SoS_dis-tribution$^{(N)}$".

Preferably, the employed deep neural networks (denoted as DNN$_x$ above) are Unet-like convolutional neural networks or ViT-like transformers (Vision transformers), as exemplarily described in the following publication which is incorporated by reference herewith: Dosovitskiy, A., Beyer, L., Kolesnikov, A., Weissenborn, D., Zhai, X., Unterthiner, T., Dehghani, M., Minderer, M., Heigold, G., Gelly, S., Uszkoreit, J. and Houlsby, N. An Image is Worth 16x16 Words: Transformers for Image Recognition at Scale. arXiv:2010.11929 (2020).

Alternatively, two baseline deep neural networks can be defined, which are simpler and less potent that the DeepOPUS network described above:

- a so-called DeepMB network, which can learn and subsequently infer the mapping:
  (OA_sinogram, SoS_distribution) $\rightarrow_{DeepMB}$ (OA_image)

- a so-called DeepUS network, which can learn and subsequently infer the mapping:
  (US_sinogram, SoS_distribution) $\rightarrow_{DeepUS}$ (US_image)

DeepMB

Preferably, the DeepMB implementation includes correctly modeling, learning and inferring a mapping between optoacoustic signals (sinogram(s)), on the one hand, and an optoacoustic image and a speed of sound (SoS) distribution, on the other hand: {optoacoustic signals} $\rightarrow$ {optoacoustic image, speed of sound distribution}.

As DeepMB is applied to optoacoustic image reconstruction only, it can be - at least to some extent - considered as a special case of the DeepOPUS implementation, which is applied to both optoacoustic and ultrasonic image reconstruction. Therefore, the above explanations regarding the DeepOPUS implementation apply accordingly to the DeepMB implementation.

Further advantages, features and examples of the present disclosure invention will be apparent from the following description of following figures:

Fig. 1    shows a schematic of an example of a DeepMB pipeline for training, in vivo imaging and evaluation;

Fig. 2    shows examples of an in vivo test dataset for different anatomical locations;

Fig. 3    shows data residual norms of optoacoustic images from deep model-based (DeepMB), model-based (MB), and backprojection (BP) reconstruction;

Fig. 4    shows a schematic representation to illustrate an improved US image reconstruction of reflective interfaces with the DeepOPUS implementation;

Fig. 5    shows a schematic representation to illustrate an improved OA image reconstruction (initial pressure distribution) with the DeepOPUS implementation; and

Fig. 6    shows a schematic representation to illustrate an improved reconstruction of the speed of sound distribution with the DeepOPUS implementation.

DeepMB

In the following, examples of a preferred deep-learning model-based optoacoustic reconstruction framework for the DeepMB implementation are shown. Advantageously, an image quality indistinguishable from state-of-the-art iterative model-based reconstructions at speeds enabling live imaging (100 fps or <10 ms/image, versus 30-60 s/image for iterative model-based reconstruction) can be obtained. Further, DeepMB overcomes the shortcomings of previous deep-learning reconstruction approaches to generalize from preferably synthetic training data to experimental test data by training on optoacoustic signals that are preferably synthesized from a publicly available dataset of real-world images, while using as ground-truth the optoacoustic images generated via model-based reconstruction of the corresponding signals. This training scheme enables DeepMB to learn an accurate and universally applicable model-based optoacoustic reconstruction operator. Further, DeepMB supports dynamic adjustments of the SoS parameter during imaging, which enables the reconstruction of in-focus images for arbitrary tissue types. In contrast to other attempts at applying deep-learning to optoacoustic reconstruction, DeepMB is directly compatible with state-of-the-art clinical MSOT (multi-spectral optoacoustic tomography) scanners because it supports high throughput data acquisition (sampling rate: 40 MHz; number of transducers: 256) and large image sizes (416×416 pixels). With DeepMB, clinical MSOT can provide high quality feedback during live imaging and thus facilitate advanced dynamic imaging applications. MSOT is a high-resolution functional imaging modality that can non-invasively quantify a broad range of pathophysiological phenomena by accessing the endogenous contrast of chromophores in tissue. For details, see e.g. Taruttis, A. and V. Ntziachristos, Advances in real-time multispectral optoacoustic imaging and its applications. Nature Photonics, 2015. 9(4): p. 219-227.

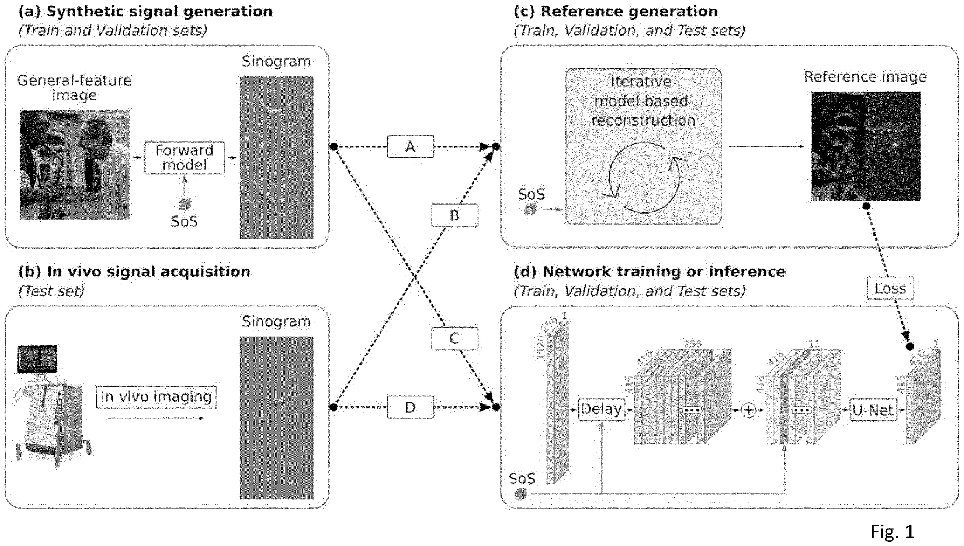

Figure 1 shows a schematic of a preferred DeepMB pipeline for training, in vivo imaging and evaluation. (a) Real-world images, preferably obtained from a publicly available dataset, are used to generate synthetic sinograms by applying an accurate physical forward model of the scanner. SoS denotes the speed-of-sound. (b) In vivo sinograms are acquired from diverse anatomical locations in patients. (c) Optoacoustic images are reconstructed via iterative model-based reconstruction for the purpose of generating reference images for either the synthetic dataset (A) or the in vivo dataset (B). (d) Network training is conducted by using the synthetic data as sets for training and validation (C), while the in vivo data constitutes the test set (D). A domain transformation is first applied to the input sinograms via a delay operation to map the time samples values into the image space. The SoS is then one-hot encoded and concatenated as additional channels. A U-Net convolutional neural network is subsequently applied to the channel stack to regress the final image. The loss is calculated between the network output and the corresponding reference image (see below for further details about the network training).

Preferably, the framework can be applied to a modern handheld optoacoustic scanner, in particular an MSOT scanner, e.g. MSOT Acuity Echo (iThera Medical GmbH, Munich, Germany), with SoS values ranging from 1475 m/s to 1525 m/s in steps of 5 m/s.

Preferably, DeepMB is trained using input sinograms synthesized from general-feature images, e.g. photographs of real-world or everyday situations, to facilitate the learning of an unbiased and universally applicable reconstruction operator. These sinograms are preferably generated by employing a diverse collection of publicly available real-world images as initial pressure distributions and simulating thereof the signals recorded by the acoustic transducers with an accurate physical model of the considered scanner (Figure 1a).

Preferably, the SoS values for the forward simulations are drawn uniformly at random from the considered range for each image. Ground-truth images for the synthesized sinograms are computed via model-based reconstruction (Figure 1c).

Figure 1d shows a preferred deep neural network architecture of DeepMB, which inputs a sinogram (either synthetic or in vivo) and a SoS value and outputs the final reconstructed image. The underlying design is preferably based on the U-net architecture augmented with two extensions that were found to be advantageous for the network to learn and express the effects of the different input SoS values onto the reconstructed images: (1) all signals are were mapped from the input sinogram to the image domain with a delay operation based on the given input SoS value and (2) the input SoS value (one-hot encoded and concatenated as additional channels) is passed to the trainable convolutional layers of the network. A detailed description of the network training is given below.

After training, the applicability of DeepMB to clinical data can be tested with a diverse dataset of in vivo sinograms acquired by scanning patients at different anatomical locations each (Figure 1b). The corresponding ground-truth images of the acquired in vivo test sinograms can be obtained analogously to the training data via model-based reconstruction. Preferably, and inference time of less than 10 ms per sample can be achieved on a modern GPU (GeForce RTX 3090).

Handheld MSOT imaging system

Preferably, a handheld MSOT scanner system is used, which is equipped with a multi-wavelength laser that illuminates tissues with short laser pulses (<10 ns) at a repetition rate of 25 Hz. Preferably, the scanner features a custom-made ultrasound detector (IMASONIC SAS, Voray-sur-l'Ognon, France) with the following characteristics: Number of piezoelectric elements: 256; Concavity radius: 4 cm; Angular coverage: 125°; Central frequency: 4 MHz. Parasitic noise generated by light-transducer interference is reduced via optical shielding of the matching layer, yielding an extended 120% frequency bandwidth. The raw channel data for each optoacoustic scan is preferably recorded with a sampling frequency of 40 MHz during 50.75 $\mu$s, yielding a sinogram of size 2030×256 samples. Co-registered B-mode ultrasound images can also be acquired and interleaved at approximately 6 Hz for live guidance and navigation. During imaging, optoacoustic backprojection images as well as B-mode ultrasound images can be displayed in real time on the scanner monitor for guidance.

Acquisition of in vivo test sinograms

To collect in vivo data for DeepMB evaluation six healthy volunteers were scanned. The involved participants were three females and three males, aged from 20 to 36 years (mean age: 28.3±5.7). Self-assessed skin color according to the Fitzpatrick scale was type II (2 participants), type III (3 p.), and type IV (1 p.). Self-assessed body type was ectomorph (2 p.), mesomorph (3 p.), and endomorph (1 p.).

For each participant, between 25 and 29 different combinations of anatomical locations and probe orientations were scanned: biceps, thyroid, carotid, calf (each left/right and transversal/longitudinal), elbow, neck, colon (each left/right), and breast (each left/right and top/bottom, female participants only). For each combination of anatomical location and probe orientation, we conducted between one and four acquisitions. During each acquisition, sinograms for approximately 10 s at wavelengths cyclically iterating from 700 to 980 nm in steps of 10 nm were recorded. Then, per acquisition, the 29 consecutively acquired sinograms were selected for which minimal motion in the interleaved ultrasound images was observed, amounting to a total of 4814 in vivo test sinograms.

Finally, all selected in vivo sinograms were band-pass filtered between 100 kHz and 12 MHz to remove frequency components beyond the transducer bandwidth and cropped the first 110 time samples to remove device-specific noise present at the beginning of the sinograms.

Determination of the SoS values

To evaluate DeepMB reconstructions under both in-focus and out-of-focus conditions, the SoS value of all in vivo test scans can be manually tuned. Preferably, a SoS step size of 5 m/s can be used to enable SoS adjustments slightly below the system spatial resolution (approximatively 200 $\mu$m). It was found that the range of optimal SoS values is approximately 1475-1525 m/s for the in vivo dataset, and therefore the same range can be used to define the supported input SoS values of the DeepMB network.

For each scan, the SoS value that resulted in the most well-focused reconstructed image was manually selected. To speed up tuning, the optimal SoS values was selected based on approximate and high-frequency-dominated reconstructions that was computed by applying the transpose model of the system to the recorded sinograms. Furthermore, the SoS was tuned only for scans at 800 nm and adopted the values for all scans at other wavelengths acquired at the time exploiting their spatial co-registration due to the absence of motion (see previous sections for details).

Synthesis of sinograms for training and validation

For network training and validation, optoacoustic sinograms are preferably synthesized with an accurate physical forward model of imaging process that incorporates the total impulse response of the system, parametrized by a SoS value drawn uniformly at random from the range (1475-1525 m/s) with step size 5 m/s. Real-world images serving as initial pressure distributions for the forward simulations can be randomly selected from the publicly available PASCAL Visual Object Classes Challenge 2012 (VOC2012) dataset, converted to mono-channel grayscale, and resized to 416×416 pixels. After the application of the forward model, each synthesized sinogram can be scaled by a factor drawn uniformly at random from the range (0-450) to match the dynamic range of in vivo sinograms.

Image reconstruction

To generate ground-truth optoacoustic images, all sinograms (synthetic as well as in vivo) were reconstructed via iterative-model-based. Preferably, Shearlet $L^1$ was used to tackle the ill-posedness of the inverse problem. Shearlet $L^1$ regularization is a convex relaxation of Shearlet sparsity, which can reduce limited-view artifacts in reconstructed images, because Shearlets provide a maximally-sparse approximation of a larger class of images (known as cartoon-like functions) with a provably optimal encoding rate, see e.g. Kutyniok, G. and W.-Q. Lim, Compactly supported shearlets are optimally sparse. Journal of Approximation Theory, 2011. 163(11): p. 1564-1589.

The optimal pressure field to find is characterized as

$$p_0 := \text{argmin}_{p \geq 0} \|M_{SoS}\, p - s\|_2 + \lambda \|SH(p)\|_1,$$

where $p_0$ is the reconstructed image, $M_{SoS}$ is the forward model of the imaging process for the selected reconstruction SoS, s is the input sinogram, $\lambda$ is the regularization parameter tuned via an L-curve, $SH$ is the Shearlet transform, and $\|...\|_n$ is the n-norm. The minimization problem is preferably solved via bound-constrained sparse reconstruction by separable approximation, see e.g. Wright, S.J., R.D. Nowak, and M.A.T. Figueiredo, Sparse Reconstruction by Separable Approximation. IEEE Transactions on Signal Processing, 2009. 57(7): p. 2479-2493 and Chartrand, R. and B. Wohlberg. Total-variation regularization with bound constraints. in 2010 IEEE International Conference on Acoustics, Speech and Signal Processing. 2010. All images can be reconstructed, e.g., with a size of 416×416 pixels and a field of view of 4.16×4.16 cm$^2$. For comparison purposes, all images can also be reconstructed using the backprojection formula, see e.g. Kunyansky, L.A., Explicit inversion formulae for the spherical mean Radon transform. Inverse Problems, 2007.

23(1): p. 373-383 and Kuchment, P. and L. Kunyansky, Mathematics of Photoacoustic and Thermoacoustic Tomography, in Handbook of Mathematical Methods in Imaging, O. Scherzer, Editor. 2011, Springer New York: New York, NY. p. 817-865.

Network training

DeepMB can be trained - either on synthetic or in vivo data - for example, for 300 epochs using stochastic gradient descent with batch size=4, learning rate=0.01, momentum=0.99, and per epoch learning rate decay factor=0.99. The network loss can be calculated as the mean square error between the output image and the reference image. The final model was selected based on the minimal loss on the validation dataset.

To facilitate training, all input sinograms can be scaled scaled by $K=450^{-1}$ to ensure that their values never exceed the range (-1 to 1). The same scaling factor can also be applied to all target images. Furthermore, the square root can be applied to all target reference images used during training and validation to reduce the network output values and limit the influence of high intensity pixels during loss calculation. When applying the trained network on in vivo test data, inferred images can be first squared and then scaled by $K^{-1}$, to revert the preprocessing operation.

When training on synthetic data to build the standard DeepMB model, for example 8000 sinograms can be used as train split and 2000 sinograms as validation split. An alternative scenario involving training on in vivo data to build the DeepMBinvivo models can be carried out as described hereafter: six different permutations can be conducted, with a 4/1/1 participants division between the train, validation, and test splits, respectively, each participant being once and only once part of the validation and test splits.

The DeepMB network is preferably based upon the U-Net architecture, preferably with a depth of 5 layers and a width of 64 features. The kernel and padding size are preferably (3, 3) and (1, 1), respectively. Preferably, biases are accounted for, and the final activation is the absolute value function.

Data residual norm

To quantify the image fidelity of reconstructions from DeepMB, model-based, or backprojection, the data residual norm R can be evaluated, which is defined as

$$R := \|M_{SoS}\, p_0 - s\|^2_2 / \|s\|^2_2,$$

where $p_0$ is the reconstructed image, $M_{SoS}$ is the forward model from model-based reconstruction, s is the input sinogram, and $\|...\|_2$ is the 2-norm. To enable a meaningful comparison between backprojection on one hand, versus non-negative model-based and DeepMB on the other hand, negative pixel values can be set to zero prior to residual calculation for backprojection images, to constrain the solution space to be analogous for all reconstruction methods. All images can be individually scaled using the linear degree of freedom in reconstructed optoacoustic image so that their data residual norms are minimal.

For evaluation of in-focus images, data residual norms can be calculated for model-based, DeepMB, and backprojection reconstructions with the optimal SoS values, for all 4814 samples from the in vivo test set. For evaluation of out-of-focus images, data residuals can be calculated for model-based and DeepMB reconstructions with all 11 SoS values, for a subset of 638 randomly selected in vivo samples.

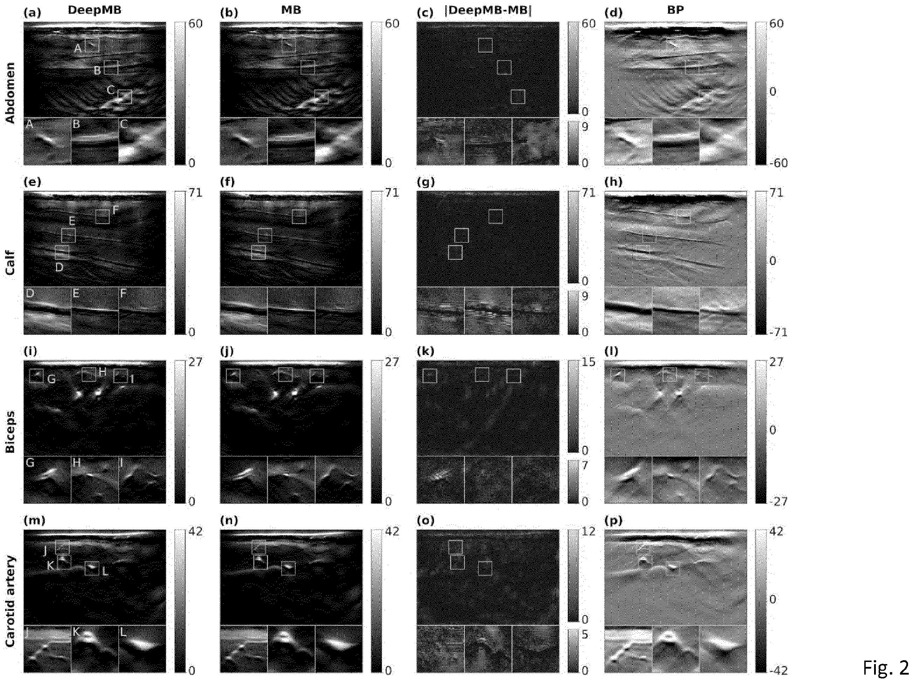

Figure 2 shows representative examples of the in vivo test dataset for different anatomical locations (abdomen: a-d, calf: e-h, biceps: i-l, carotid artery: m-p). The two leftmost columns correspond to deep model-based (DeepMB) and model-based (MB) reconstructions. The third column represents the absolute difference between DeepMB and model-based images. The rightmost column depicts the backprojection (BP) reconstructions. The shown field of view is $4.16 \times 2.80$ cm$^2$, each enlarged region is $0.41 \times 0.41$ cm$^2$.

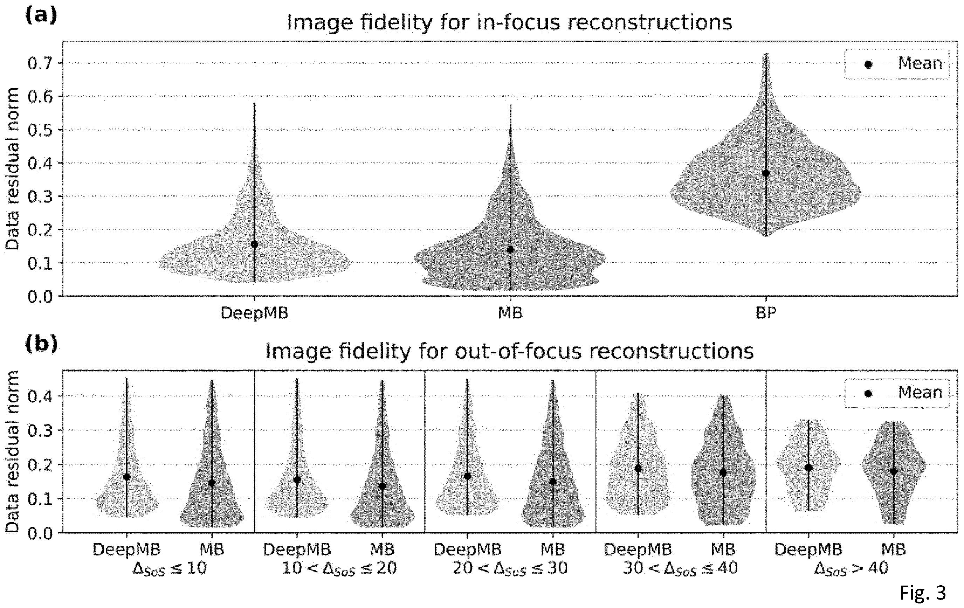

Figure 3 shows data residual norms of optoacoustic images from deep model-based (DeepMB), model-based (MB), and backprojection (BP) reconstruction. (a) Data residual norms of in-focus images reconstructed with optimal speed of sound (SoS) values, on all 4814 samples from the in vivo test set. (b) Data residual norms of out-of-focus images reconstructed with sub-optimal SoS values, on a subset of 638 samples. The five sub-panels depict the effect of SoS mismatch via gradual increase of the offset $\Delta$SoS in steps of 10 m/s.

Qualitative evaluation

To evaluate the capacity of DeepMB to reconstruct high-quality images, all DeepMB images from the clinical dataset (Figure 1b) are compared to their corresponding model-based reference images (Figure 1c). Figure 2 illustrates that the DeepMB reconstructions (Figure 2 a, e, i, m) were systematically nearly-indistinguishable from the model-based references (Figure 2 b, f, j, n), with no noticeable failures, outliers, or artifacts for any of the participants, anatomies, probe orientations, SoS values, or laser wavelengths. The similarity between the DeepMB and model-based images is also confirmed by their negligible pixel-wise absolute differences (Figure 2 c, g, k, o). The zoomed region G in Figure 2 i-k depicts one of the largest observed discrepancies between the DeepMB and model-based reconstructions, which manifests as minor blurring, showing that the DeepMB image is only marginally affected by these errors. In comparison, backprojection images (Figure 2 d, h, I, p) exhibit notable differences from the reference model-based images and suffer from reduced spatial resolution and physically-nonsensical negative initial pressure values.

Quantitative evaluation

To quantify the image fidelity of DeepMB reconstructions, the data residual norm was calculated for all in vivo test images. The data residual norm measures the fidelity of a reconstructed image by computing the mismatch between the image and the corresponding recorded acoustic signals with regard to the accurate physical forward model of the used scanner, and is provably minimal for model-based reconstruction. The data residual norm can also be calculated for all model-based and backprojection reconstructions, for comparison purposes.

First, the calculated data residual norms of in-focus images reconstructed with optimal SoS values can be assessed to evaluate the fidelity of DeepMB images with the best possible quality (Figure 3a). Data residual norms of DeepMB images (mean = 0.156) are almost as low as the provably minimal data residual norms of model-based (MB) images (mean = 0.139), whereas the data residual norms of backprojection (BP) images (mean = 0.369) are substantially higher. The close agreement between data residual norms of DeepMB and model-based images confirms that both reconstruction approaches afford equivalent image qualities. In contrast, the data residual norms of backprojection images are markedly higher, which reaffirms the shortcomings of backprojection to accurately model the imaging process, and explains the lower image quality observed in Figure 2 d, h, I, p.

Second, the data residual norms of out-of-focus images reconstructed with sub-optimal SoS values can be assessed to evaluate the fidelity of DeepMB images during imaging applications with a priori unknown SoS (Figure 3b). Data residual norms of DeepMB images remain close to those of model-based images for all considered levels of mismatch between the optimal and the employed SoS, thus confirming that DeepMB and model-based images are similarly trustworthy independent of the selected SoS.

Advantages of synthesized training data

Finally, to assess the advantage of using synthesized training data for DeepMB to learn an accurate and general reconstruction operator, alternative DeepMB models can be trained on in vivo data instead of synthetic data. These models, referred to as DeepMBinvivo, inferred images with similar data residual norms (mean=0.155) as the standard DeepMB model. However, DeepMBinvivo images may contain visible artifacts, either at the left or right image borders, or in regions showing strong absorption at the skin surface. On the other hand, no artifacts are observed with the preferred training strategy of DeepMB (using synthesized training data), even when reducing the size of the synthetic training set from 8000 to 3500 to match the reduced amount of available in vivo training data.

As demonstrated above, DeepMB achieves three seminal features compared to previous approaches: Accurate generalization to in vivo measurements after training on synthesized sinograms that were derived from real-world images, dynamic adjustment of the reconstruction SoS during imaging, and compatibility with the data rates and image sizes of modern MSOT scanners. DeepMB therefore enables dynamic-imaging applications of optoacoustic tomography and deliver high-quality images to clinicians in real-time during examinations, furthering the clinical translation of this technology and leading to more accurate diagnoses and surgical guidance.

DeepMB is preferably trained on synthesized sinograms from real-world images, instead of in vivo images, because these synthesized sinograms afford a large training dataset with a versatile set of image features, allowing DeepMB to accurately reconstruct images with diverse features. In particular, such general-feature training datasets reduce the risk of encountering out-of-distribution samples (test data with features that are not contained in the training dataset) when applying the trained model to in vivo scans. In contrast, training a model on in vivo scans may systematically introduce the risk of overfitting to specific characteristics of the training samples and can lead to decreased image quality for never-seen-before scans that may involve different anatomical views, body types, skin colors, or disease states. It can be observed that alternative DeepMB in vivo models trained on in vivo data fail to adequately generalize to some in vivo test scans and introduce artifacts within the reconstructed images. Furthermore, using synthesized data instead of in vivo data alleviates the training of new DeepMB models because it obviates the need for recruiting and scanning a cohort of participants. Instead, training data can be automatically generated and used to straightforwardly obtain specifically-trained DeepMB models for new scanners or different reconstruction parameters.

Accurate generalization from synthesized training to in vivo test data is possible with DeepMB because the underlying inverse problem to solve (that is, regularized model-based reconstruction) is well-posed; for each input sinogram there is a unique and stable solution (i.e., the reconstructed image). Therefore, the network can learn a data transform that is agnostic to specific characteristics of the ground-truth images during training and generalizes to images with any content (be it synthesized or in vivo). In contrast, previous deep-learning-based optoacoustic reconstruction approaches were limited in their ability to generalize from synthesized training data to in vivo test data because the underlying inverse problems were ill-posed. More precisely, the targets used during the training of these deep neural networks were the true synthetic initial pressure images (left side in Figure 1a), containing image information not available in the input sinogram due to limited angle acquisition, measurement noise, or finite transducer bandwidth. To restore the missing information, these deep neural network models were required to incorporate information from the training data manifold, which limited their generalization and provides a likely explanation for the rudimentary image quality previously reported for in vivo measurements.

The disclosed DeepMB methodology to increase the speed of iterative model-based reconstruction is also applicable to other optoacoustic reconstruction approaches. For instance, frequency-band model-based reconstruction or Bayesian optoacoustic reconstruction can disentangle structures of different physical scales and quantifying reconstruction uncertainty, respectively, but their long reconstruction times currently hinder their use in real-time applications. The methodology of DeepMB could also be exploited to accelerate parametrized (iterative) inversion approaches for other imaging modalities, such as ultrasound, X-ray computed tomography, magnetic resonance imaging, or, more generally, for any parametric partial differential equation. In conclusion, DeepMB is a fully operational software-based prototype for real-time model-based optoacoustic image reconstruction, which can be e.g. embedded into the hardware of a modern MSOT scanner to use DeepMB for real-time imaging in clinical applications.

DeepOPUS

As DeepMB is applied to optoacoustic image reconstruction only, it can be - at least to some extent - considered as a special case of the DeepOPUS implementation, which is applied to both optoacoustic and ultrasonic image reconstruction.

Therefore, the above explanations regarding the DeepMB implementation apply accordingly to the DeepOPUS implementation.

Further, DeepOPUS preferably relates to a deep learning solution for simultaneous reconstruction of co-registered optoacoustic (OA) and ultrasonic (US) images, so that synergistic effects between these two imaging modalities can be used, i.e. US image reconstruction is improved by considering information from OA data and/or OA image(s) and OA image reconstruction is improved by considering information from US data and/or US image(s). This will be explained in more detail in the following.

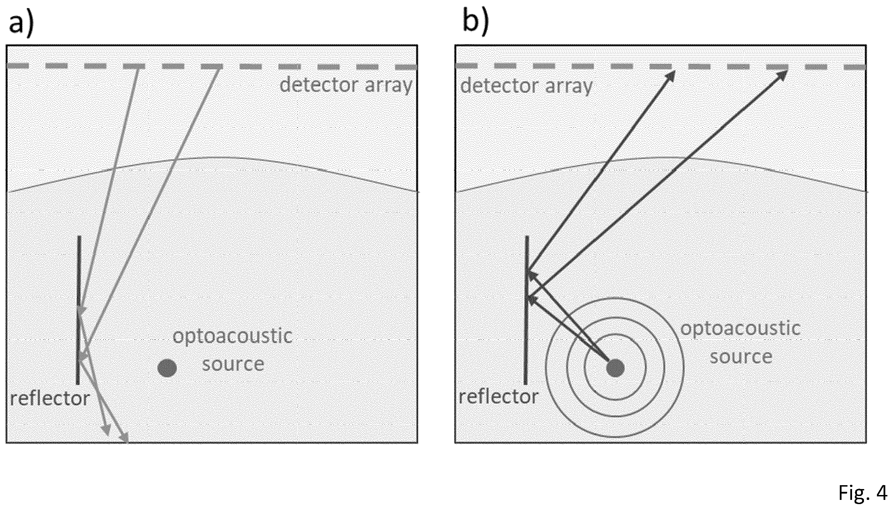

Figure 4 shows a schematic representation to illustrate an improved US image reconstruction of reflective interfaces. As illustrated in Figure 4a, a reflector within a sample cannot be detected by reflection ultrasound tomography, since signals are not reflected back to the transducer array, which acts both as a transmitter of ultrasound waves and a detector for reflected ultrasound waves. As illustrated in Figure 4b, an optoacoustic source within the sample acts like a probe (emitting ultrasound waves) for the reflector, enabling tomographic reconstruction.

As a result, DeepOPUS allows for an improved reconstruction of $\Gamma$ via the mapping (US data, OA data, $p_0$, SoS) $\mapsto \Gamma$, wherein $p_0$ acts as additional probe from within the sample, as illustrated in Figure 4. In other words, knowing the pressure waves that are generated by the optoacoustic sources (given by $p_0$), one can recover additional information about the location of reflective interfaces from the acquired (and potentially reflected) OA signals for an improved reconstruction of ultrasonic images.

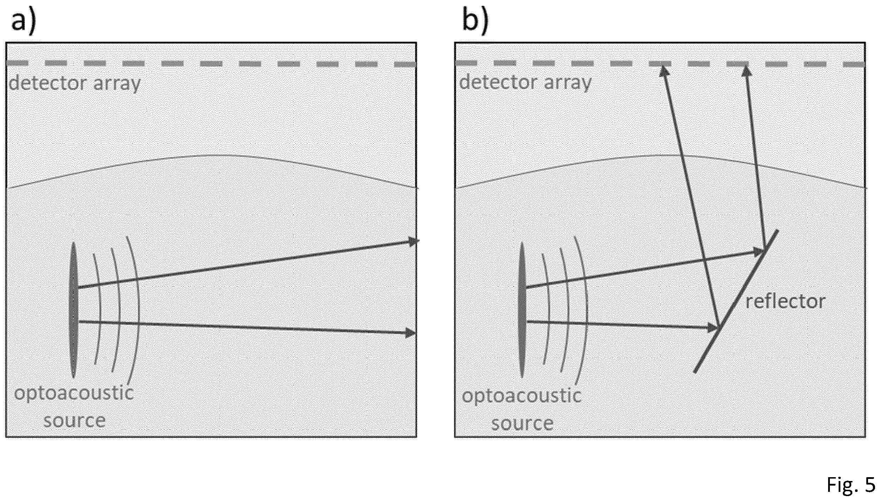

Figure 5 shows a schematic representation to illustrate an improved OA image reconstruction (initial pressure distribution). As illustrated in Figure 5a, detector arrays with limited angular coverage cannot detect optoacoustic sources that are elongated perpendicular to the array, since the generated waves are directional and do not hit the detector. As illustrated in Figure 4b, knowledge of reflectors that reflect signals generated by elongated structures towards the detector allows detection of such structures.

As a result, DeepOPUS allows for an improved reconstruction of $p_0$ via the mapping (US data, OA data, SoS, $\Gamma$) $\mapsto p_0$, wherein reflections are removed via knowledge of $\Gamma$, limited angular coverage is reduced by signals reflected back to transducers via knowledge of $\Gamma$, as illustrated in Figure 5. In other words: Optoacoustic waves that would normally not arrive at the detector array due to the limited angular coverage can potentially be reflected to the detectors at reflective interfaces (as given by $\Gamma$). Acquisition of such signals in combination with knowledge of the location of reflective interfaces allows to improve the reconstruction and mitigate limited view artefacts.

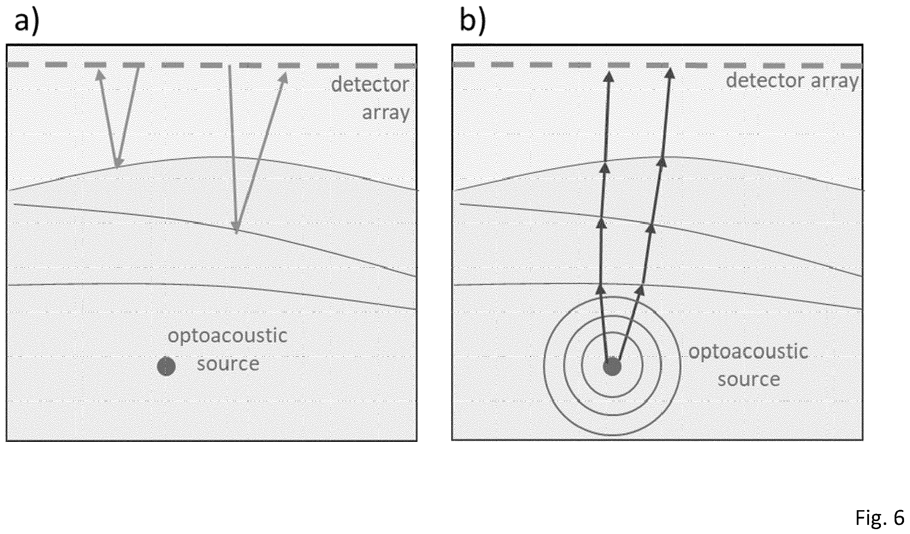

Figure 6 shows a schematic representation to illustrate an improved reconstruction of the speed of sound distribution. As illustrated in Figure 6a, reflection ultrasound data is not suitable for reconstruction of the speed of sound distributions due to its limited information about times of flight through tissue layers. As illustrated in Figure 6b, optoacoustic sources can serve as probes for transmission ultrasound data that simplify speed of sound reconstruction significantly.

As a result, DeepOPUS allows for an improved reconstruction of SoS in reflection mode US with the help of OA via the mapping (US data, OA data, $\Gamma$, $p_0$) $\mapsto$ SoS, wherein OA data is partial transmission data. In other words: Although reconstruction of the speed of sound distribution from reflective US data (i.e., waves that are at least once reflected within the sample) alone is highly ill-posed and, therefore, only achievable with rudimentary quality, knowledge of the locations of optoacoustic sources (as given by $p_0$) that send acoustic waves through the tissue towards the detector without being reflected provides transmission data from which the reconstruction of the speed of sound distribution is possible with much higher quality and reliability.

**Claims**

1. A method for training an artificial neural network for reconstructing optoacoustic and/or ultrasonic images from signals generated by an imaging apparatus for optoacoustic and/or ultrasonic imaging, the method comprising:

   a) providing a model of the imaging apparatus, the model characterizing a relation between i) a spatial distribution of acoustic sources emitting and/or reflecting acoustic waves and ii) signals generated by detection elements of the imaging apparatus upon detecting the acoustic waves,
   b) providing several training signal sets, each training signal set comprising a plurality of training signals which were i) generated by the imaging apparatus upon imaging objects and/or ii) obtained by simulating an imaging of objects by the imaging apparatus based on the model of the imaging apparatus,
   c) reconstructing, based on the model of the imaging apparatus, several training image data sets from the training signal sets, each training image data set comprising image data relating to an optoacoustic and/or ultrasonic image of an object, and
   d) training the artificial neural network, which comprises an input layer and an output layer, the training comprising i) inputting the training signal sets at the input layer, ii) obtaining, for each inputted training signal set, an output image data set which is outputted at the output layer, and iii) comparing each output image data set with the training image data set which was reconstructed from the respectively inputted training signal set.

2. The method according to claim 1, the model characterizing at least one of the following: i) a propagation of the acoustic waves from the acoustic sources towards the detection elements, ii) a response of the detection elements upon detecting the acoustic waves, and/or iii) a noise of the imaging apparatus.

3. The method according to claim 2, wherein characterizing the propagation of the acoustic waves includes at least one of the following: i) an acoustic wave propagation model, which is the same for the propagation of both emitted optoacoustic waves and reflected ultrasound waves, ii) a propagation of the acoustic waves through a medium with an inhomogeneous speed of sound distribution, and/or iii) a reflection of the acoustic waves at one or more reflective interfaces in the medium.

4. The method according to any one of the preceding claims, wherein at least some of the training signal sets comprise training signals which were obtained, in particular synthesized, by simulating imaging of objects by the imaging apparatus based on i) the model of the imaging apparatus and ii) initial images of objects which were obtained by any imaging apparatus.

5. The method according to any one of the preceding claims, wherein reconstructing a training image data set ($x^*$) from a training signal set ($s$) comprises:

   i) calculating, based on the model ($M$) of the imaging apparatus, several prediction signal sets ($M(x)$) from several varying image data sets ($x$),
   ii) calculating, for each of the varying image data sets ($x$), a first distance metric ($d(M(x), s)$) between the respective prediction signal set ($M(x)$) and the training signal set ($s$), and
   iii) determining the image data set ($x^*$) for which the first distance metric ($d(M(x), s)$) between the respective prediction signal set ($M(x)$) and the training signal set ($s$) exhibits a minimum,

   wherein the reconstructed training image data set is the determined image data set ($x^*$).

6. The method according to any one of the claims 1 to 4, wherein reconstructing at least one training image data set ($x^*_{OA}$, $X^*_{US}$, $c^*$) from at least one training signal set ($s_{OA}$, $s_{US}$) comprises:

   i) calculating, based on the model ($M_c$) of the imaging apparatus taking into account a propagation of the acoustic waves through a medium with a, in particular pre-defined or reconstructed, speed of sound distribution (c), several prediction signal sets ($M_c(x_{OA}, x_{US})$) from several varying image data sets ($x_{OA}, x_{US}$),

ii) calculating, for each of the varying image data sets ($x_{OA}$, xus), a second distance metric ($d(M_c(x_{OA}, x_{US})$, ($s_{OA}, s_{US}$)) between the respective prediction signal set ($M_c(x_{OA}, x_{US})$) and the training signal set ($s_{OA}, s_{US}$), and

iii) determining at least one image data set ($x^*_{OA}, X^*_{US}, c^*$) for which the second distance metric ($d(M_c(x_{OA}, x_{US})$, ($s_{OA}, s_{US}$)) between the respective prediction signal set ($d(M_c(x_{OA}, x_{US})$) and the at least one training signal set ($s_{OA}, s_{US}$) exhibits a minimum,

wherein the at least one training image data set is the at least one determined image data set ($x^*_{OA}, X^*_{US}, c^*$).

7. The method according to claim 5 or 6, wherein comparing the output image data set with the respective training image data set ($x^*_{OA}, X^*_{US}, c^*$) comprises determining a loss function which is given by:

- a third distance metric, in particular a means squared error, between the output image data set, on the one hand, and the respective training image data set ($x^*_{OA}, x^*_{US}$) and speed of sound distribution ($c^*$) reconstructed from the respective training signal set, on the other hand, and/or
- the first and/or second distance metric which is applied to the output image data set.

8. The method according to any one of the preceding claims, wherein the at least one artificial neural network is given by i) a single deep neural network or ii) a cascade of multiple (N) deep neural networks.

9. The method according to any one of the preceding claims, wherein the training comprises (one-step process) i) inputting the training signal sets ($s_{OA}, s_{US}$) at the input layer, ii) obtaining, for each inputted training signal set ($s_{OA}$, sus), both the output image data set ($x_{OA}, x_{US}$) and an output speed of sound distribution (c) which are outputted at the output layer, and iii) comparing each output image data set ($x_{OA}, x_{US}$) and output speed of sound distribution (c) with the training image data set ($x^*_{OA}, x^*_{US}$) and, respectively, a training speed of sound distribution ($c^*$) which were reconstructed from the respectively inputted training signal set ($s_{OA}, s_{US}$).

10. The method according to any one of the preceding claims, wherein the training comprises (two-step process),

- i) inputting the training signal sets ($s_{OA}, s_{US}$) at the input layer, ii) obtaining, for each inputted training signal set ($s_{OA}, s_{US}$), an output speed of sound distribution (c) which is outputted at the output layer, and iii) comparing each output speed of sound distribution (c) with a training speed of sound distribution ($c^*$) which was reconstructed from the respectively inputted training signal set ($s_{OA}$, sus), and subsequently
- i) inputting the training signal sets ($s_{OA}$, sus) and the output speed of sound distribution (c) at the input layer, ii) obtaining, for each inputted training signal set ($s_{OA}$, sus) and output speed of sound distribution (c), the output image data set ($x_{OA}, x_{US}$) which is outputted at the output layer, and iii) comparing each output image data set ($x_{OA}, x_{US}$) with the training image data set ($x^*_{OA}, x^*_{US}$) which was reconstructed from the respectively inputted training signal set ($s_{OA}, s_{US}$).

11. A method for reconstructing an optoacoustic and/or ultrasonic image from a set of signals generated by an imaging apparatus for optoacoustic and/or ultrasonic imaging, the method comprising:

- inputting the set of signals at an input layer of the artificial neural network which has been trained by the method according to any one of the preceding claims, and
- obtaining at least one optoacoustic and/or ultrasonic image which is outputted at an output layer of the trained artificial neural network.

12. A method for optoacoustic and/or ultrasonic imaging comprising:

- irradiating an object with electromagnetic and/or acoustic waves and generating a set of signals by detecting acoustic waves emitted and/or reflected by the object in response thereto by means of an imaging apparatus for optoacoustic and/or ultrasonic imaging, and
- reconstructing an optoacoustic and/or ultrasonic image of the object from the set of signals by the method according to the previous claim.

13. A system for optoacoustic and/or ultrasonic imaging comprising

- an imaging apparatus for optoacoustic and/or ultrasonic imaging, the imaging apparatus comprising an irradiation device configured to irradiate an object with electromagnetic and/or acoustic waves and a detection device configured to generate a set of signals by detecting acoustic waves emitted and/or reflected by the object in response to irradiating the object with the electromagnetic and/or acoustic waves, and
- a processor configured to reconstruct an optoacoustic and/or ultrasonic image of the object from the set of signals by inputting the set of signals at an input layer of an artificial neural network which has been trained by the method according to any one of the claims 1 to 10, and obtaining at least one optoacoustic and/or ultrasonic image which is outputted at an output layer of the trained artificial neural network.

14. A computer program product causing a computer, computer system and/or distributed computing environment to execute the method according to any one of claims 1 to 10 and/or claim 11 and/or claim 12.

**(a) Synthetic signal generation**
*(Train and Validation sets)*

General-feature image

Forward model

SoS

Sinogram

**(c) Reference generation**
*(Train, Validation, and Test sets)*

Iterative model-based reconstruction

SoS

Reference image

**(b) In vivo signal acquisition**
*(Test set)*

In vivo imaging

Sinogram

**(d) Network training or inference**
*(Train, Validation, and Test sets)*

Delay

SoS

U-Net

Loss

A

B

C

D

Fig. 1

Fig. 2

**(a)** Image fidelity for in-focus reconstructions

**(b)** Image fidelity for out-of-focus reconstructions

Fig. 3

Fig. 4

EP 4 285 835 A1

EP 4 285 835 A1

a) / b)

Fig. 5

a)

detector array

optoacoustic source

b)

detector array

optoacoustic source

EP 4 285 835 A1

Fig. 6

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/177461 A1 (BELL MUYINATU [US] ET AL) 28 June 2018 (2018-06-28) * paragraphs [0003] – [0006], [0010], [0011], [0015], [0016], [0032] – [0038], [0044] – [0057], [0065], [0067]; claims 1,6-8; figures 1-10 * | 1-14 | INV. A61B8/08 A61B5/00 |
| X | AGRAWAL SUMIT ET AL: "Modeling combined ultrasound and photoacoustic imaging: Simulations aiding device development and artificial intelligence", PHOTOACOUSTICS, vol. 24, 1 December 2021 (2021-12-01), page 100304, XP055968478, ISSN: 2213-5979, DOI: 10.1016/j.pacs.2021.100304 * section 2: Methodology section 3.6; USPA simulation aided artificail intelligence (AI) for PAI, section 3.6.1; figures 3, 11 * * abstract * | 1-4,8, 11-14 | |
| X | LAN HENGRONG ET AL: "Reconstruct the Photoacoustic Image Based On Deep Learning with Multi-frequency Ring-shape Transducer Array", 2019 41ST ANNUAL INTERNATIONAL CONFERENCE OF THE IEEE ENGINEERING IN MEDICINE AND BIOLOGY SOCIETY (EMBC), IEEE, 23 July 2019 (2019-07-23), pages 7115-7118, XP033624504, DOI: 10.1109/EMBC.2019.8856590 [retrieved on 2019-10-02] * abstract * * section II. Method, section III. Results; figures 1-4 * | 1-8, 11-14 | TECHNICAL FIELDS SEARCHED (IPC) A61B G06T |

-/--

The present search report has been drawn up for all claims

| Place of search | Date of completion of the search | Examiner |
|---|---|---|
| Munich | 28 October 2022 | Faber-Jurk, Sonja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 22 17 7153

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/341436 A1 (PERDIOS DIMITRIS [CH] ET AL) 4 November 2021 (2021-11-04) * paragraphs [0001] - [0003], [0027] - [0036], [0077] - [0081]; claims 1-4; figures 1-8 * | 1-4, 11-14 | |

TECHNICAL FIELDS
SEARCHED     (IPC)

The present search report has been drawn up for all claims

| Place of search | Date of completion of the search | Examiner |
|---|---|---|
| Munich | 28 October 2022 | Faber-Jurk, Sonja |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................
& : member of the same patent family, corresponding
document

EPO FORM 1503 03.82 (P04C01)

page 2 of 2

## ANNEX TO THE EUROPEAN SEARCH REPORT
## ON EUROPEAN PATENT APPLICATION NO.

EP 22 17 7153

28-10-2022

| Patent document cited in search report | | Publication date | Patent family member(s) | | Publication date |
|---|---|---|---|---|---|
| US 2018177461 | A1 | 28-06-2018 | NONE | | |
| US 2021341436 | A1 | 04-11-2021 | CN | 112771374 A | 07-05-2021 |
| | | | EP | 3637099 A1 | 15-04-2020 |
| | | | ES | 2886155 T3 | 16-12-2021 |
| | | | IL | 281888 A | 31-05-2021 |
| | | | JP | 2022503946 A | 12-01-2022 |
| | | | KR | 20210069655 A | 11-06-2021 |
| | | | US | 2021341436 A1 | 04-11-2021 |
| | | | WO | 2020074181 A1 | 16-04-2020 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHOWDHURY, K.B. ; PRAKASH, J. ; KARLAS, A. ; JÜSTEL, D. ; NTZIACHRISTOS, V.** A Synthetic Total Impulse Response Characterization Method for Correction of Hand-Held Optoacoustic Images. *IEEE Trans Med Imaging,* 2020, vol. 39, 3218-3230
- **CHOWDHURY, K.B. ; BADER, M. ; DEHNER, C. ; JÜSTEL, D. ; NTZIACHRISTOS, V.** Individual transducer impulse response characterization method to improve image quality of array-based handheld optoacoustic tomography. *Opt Lett,* 2021, vol. 46, 1-4
- **DEHNER, C. ; OLEFIR, I. ; BASAK CHOWDHURY, K. ; JÜSTEL, D. ; NTZIACHRISTOS, V.** Deep learning based electrical noise removal enables high spectral optoacoustic contrast in deep tissue. *arXiv:2102.12960,* 2021
- **HAUPTMANN, A. ; LUCKA, F. ; BETCKE, M. ; HUYNH, N. ; ADLER, J. ; COX, B. ; BEARD, P. ; OURSELIN, S. ; ARRIDGE, S.** Model-Based Learning for Accelerated, Limited-View 3-D Photoacoustic Tomography. *IEEE Trans Med Imaging,* 2018, vol. 37, 1382-1393
- **DOSOVITSKIY, A. ; BEYER, L. ; KOLESNIKOV, A. ; WEISSENBORN, D. ; ZHAI, X. ; UNTERTHINER, T. ; DEHGHANI, M. ; MINDERER, M. ; HEIGOLD, G. ; GELLY, S.** An Image is Worth 16x16 Words: Transformers for Image Recognition at Scale. *arXiv:2010.11929,* 2020

- **TARUTTIS, A. ; V. NTZIACHRISTOS.** Advances in real-time multispectral optoacoustic imaging and its applications. *Nature Photonics,* 2015, vol. 9 (4), 219-227
- **KUTYNIOK, G. ; W.-Q. LIM.** Compactly supported shearlets are optimally sparse. *Journal of Approximation Theory,* 2011, vol. 163 (11), 1564-1589
- **WRIGHT, S.J. ; R.D. NOWAK ; M.A.T. FIGUEIREDO.** Sparse Reconstruction by Separable Approximation. *IEEE Transactions on Signal Processing,* 2009, vol. 57 (7), 2479-2493
- **CHARTRAND, R. ; B. WOHLBERG.** Total-variation regularization with bound constraints. *2010 IEEE International Conference on Acoustics, Speech and Signal Processing,* 2010
- **KUNYANSKY, L.A.** Explicit inversion formulae for the spherical mean Radon transform. *Inverse Problems,* 2007, vol. 23 (1), 373-383
- Mathematics of Photoacoustic and Thermoacoustic Tomography. **KUCHMENT, P. ; L. KUNYANSKY.** Handbook of Mathematical Methods in Imaging. Springer, 2011, 817-865